# EUROPEAN PATENT APPLICATION

(11) **EP 2 345 737 A1**
(43) Date of publication of application: **20.07.2011**
(21) Application number: 10150632.7
(22) Date of filing: 13.01.2010
(51) Int. Cl.: C12Q 1/68

(54) **Method for identifying inhibitors of protein translocation**

(71) Applicant: Harant, Hanna, 1140 Vienna (AT)
(72) Inventor: Harant, Hanna, 1140 Vienna (AT); Korschineck, Irina, 1060 Vienna (AT)
(74) Representative: Sonn & Partner Patentanwälte

(57) **Abstract**

The present invention relates to a method for identifying an inhibitor of protein translocation comprising the steps of:
a) providing a nucleic acid molecule encoding a polypeptide comprising a translocation signal domain and/or a transmembrane domain and having no stop codon in the reading frame of said polypeptide,
b) translating *in vitro* the nucleic acid molecule of step a) into a ribosome bound nascent polypeptide by contacting said nucleic acid molecule with an *in vitro* translation system comprising ribosomes,
c) optionally contacting the ribosome bound nascent polypeptide of step b) with a compound releasing the polypeptide from the ribosome and isolating said released polypeptide,
d) incubating at least one test compound or a library of test compounds
i) with the ribosome bound nascent polypeptide of step b) or optionally
ii) with the isolated polypeptide of step c),

e) isolating the ribosome bound nascent polypeptide or optionally isolated polypeptide of step d) and identifying the test compound bound to said polypeptide.

SEQUENCE LISTING

<110>  Harant, Hanna

<120>  Method for identifying inhibitors

<130>  R 54990

<160>  51

<170>  PatentIn version 3.5

<210>  1
<211>  7
<212>  PRT
<213>  Artificial Sequence

<220>
<223>  Peptide binding to the signal peptide of VEGF

<400>  1

Pro Leu His Ala Arg Leu Pro
1               5


<210>  2
<211>  6
<212>  PRT
<213>  Artificial Sequence

<220>
<223>  Peptide binding to the signal peptide of VEGF

<400>  2

Phe Leu Phe Arg Leu Phe
1               5


<210>  3
<211>  7
<212>  PRT
<213>  Artificial Sequence

<220>
<223>  Peptide binding to the signal peptide of VEGF

<400>  3

Pro Met Thr Ser Pro Pro Ser
1               5


<210>  4
<211>  7
<212>  PRT
<213>  Artificial Sequence

<220>
<223>  Peptide binding to the signal peptide of VEGF

<400>  4

Asn Pro Ile Ser Leu His Leu
1               5


<210>  5
<211>  7

<212>  PRT
<213>  Artificial Sequence

<220>
<223>  Peptide binding to the signal peptide of VEGF

<400>  5

Thr Ala Ser Ser Val Lys Leu
1               5


<210>  6
<211>  7
<212>  PRT
<213>  Artificial Sequence

<220>
<223>  Peptide binding to the signal peptide of VEGF

<400>  6

Ser Val Ser Gly Ser Pro Ser
1               5


<210>  7
<211>  7
<212>  PRT
<213>  Artificial Sequence

<220>
<223>  Peptide binding to the signal peptide of VEGF

<400>  7

Ser Gly Ser Ser Pro Trp His
1               5


<210>  8
<211>  7
<212>  PRT
<213>  Artificial Sequence

<220>
<223>  Peptide binding to the signal peptide of VEGF

<400>  8

His Pro Ser Ser Thr His Gln
1               5


<210>  9
<211>  7
<212>  PRT
<213>  Artificial Sequence

<220>
<223>  Peptide binding to the signal peptide of VEGF

<400>  9

Asn Leu Ser Ser Ser Trp Ile
1               5


<210>  10
<211>  7

<212>  PRT
<213>  Artificial Sequence

<220>
<223>  Peptide binding to the signal peptide of VEGF

<400>  10

Leu Thr Arg Pro Ser Pro Ser
1               5


<210>  11
<211>  7
<212>  PRT
<213>  Artificial Sequence

<220>
<223>  Peptide binding to the signal peptide of VEGF

<400>  11

Ser Pro Ser Leu Leu Pro Ala
1               5


<210>  12
<211>  7
<212>  PRT
<213>  Artificial Sequence

<220>
<223>  Peptide binding to the signal peptide of VEGF

<400>  12

Ser Ser Pro Leu Leu Gly Arg
1               5


<210>  13
<211>  7
<212>  PRT
<213>  Artificial Sequence

<220>
<223>  Peptide binding to the signal peptide of VEGF

<400>  13

Pro Val Ala Pro Ser Thr His
1               5


<210>  14
<211>  7
<212>  PRT
<213>  Artificial Sequence

<220>
<223>  Peptide binding to the signal peptide of VEGF

<400>  14

Leu Gly Ala Leu Ser His Thr
1               5


<210>  15
<211>  7

<212>  PRT
<213>  Artificial Sequence

<220>
<223>  Peptide binding to the signal peptide of VEGF

<400>  15

Ser Pro Arg Pro Ser Leu Ser
1               5


<210>  16
<211>  7
<212>  PRT
<213>  Artificial Sequence

<220>
<223>  Peptide binding to the signal peptide of VEGF

<400>  16

Pro Ala Asn Phe Gly Pro Ser
1               5


<210>  17
<211>  7
<212>  PRT
<213>  Artificial Sequence

<220>
<223>  Peptide binding to the signal peptide of VEGF

<400>  17

Leu Thr Leu Pro Asn Arg Thr
1               5


<210>  18
<211>  7
<212>  PRT
<213>  Artificial Sequence

<220>
<223>  Peptide binding to the signal peptide of VEGF

<400>  18

Ala Phe Pro Gly Met Lys Ala
1               5


<210>  19
<211>  7
<212>  PRT
<213>  Artificial Sequence

<220>
<223>  Peptide binding to the signal peptide of VEGF

<400>  19

Leu Pro Pro Leu Gln Arg Leu
1               5


<210>  20
<211>  7

<212>  PRT
<213>  Artificial Sequence

<220>
<223>  Peptide binding to the signal peptide of VEGF

<400>  20

Pro Pro Pro Phe Leu Pro Leu
1               5


<210>  21
<211>  7
<212>  PRT
<213>  Artificial Sequence

<220>
<223>  Peptide binding to the signal peptide of VEGF

<400>  21

Glu Ile Arg Thr Pro Ser Val
1               5


<210>  22
<211>  7
<212>  PRT
<213>  Artificial Sequence

<220>
<223>  Peptide binding to the signal peptide of VEGF

<400>  22

His Ala Trp Thr Phe Asn Ser
1               5


<210>  23
<211>  7
<212>  PRT
<213>  Artificial Sequence

<220>
<223>  Peptide binding to the signal peptide of VEGF

<400>  23

Ser Tyr Pro Leu Arg Pro Pro
1               5


<210>  24
<211>  7
<212>  PRT
<213>  Artificial Sequence

<220>
<223>  Peptide binding to the signal peptide of VEGF

<400>  24

Leu Pro Val Ser Ser Ser Ser
1               5


<210>  25
<211>  7

<212>  PRT
<213>  Artificial Sequence

<220>
<223>  Peptide binding to the signal peptide of VEGF

<400>  25

Leu Ala Leu Ser Ala Ala Arg
1               5


<210>  26
<211>  7
<212>  PRT
<213>  Artificial Sequence

<220>
<223>  Peptide binding to the signal peptide of VEGF

<400>  26

Ser Leu Asn Ser Asp Leu Phe
1               5


<210>  27
<211>  7
<212>  PRT
<213>  Artificial Sequence

<220>
<223>  Peptide binding to the signal peptide of VEGF

<400>  27

Ser Pro His Leu Ile Pro Ser
1               5


<210>  28
<211>  7
<212>  PRT
<213>  Artificial Sequence

<220>
<223>  Peptide binding to the signal peptide of VEGF

<400>  28

His Pro Met Phe Pro Leu Ser
1               5


<210>  29
<211>  7
<212>  PRT
<213>  Artificial Sequence

<220>
<223>  Peptide binding to the signal peptide of VEGF

<400>  29

His Pro Met Phe Trp Pro Ile
1               5


<210>  30
<211>  7

<212>  PRT
<213>  Artificial Sequence

<220>
<223>  Peptide binding to the signal peptide of VEGF

<400>  30

Trp Ala Ser Gln Ala His Pro
1               5


<210>  31
<211>  7
<212>  PRT
<213>  Artificial Sequence

<220>
<223>  Peptide binding to the signal peptide of VEGF

<400>  31

Ser Ser Thr Leu His Pro Ser
1               5


<210>  32
<211>  7
<212>  PRT
<213>  Artificial Sequence

<220>
<223>  Peptide binding to the signal peptide of VEGF

<400>  32

His Ala Leu Ser Ser Arg Thr
1               5


<210>  33
<211>  7
<212>  PRT
<213>  Artificial Sequence

<220>
<223>  Peptide binding to the signal peptide of VEGF

<400>  33

Pro His Thr Arg Gly Phe Met
1               5


<210>  34
<211>  7
<212>  PRT
<213>  Artificial Sequence

<220>
<223>  Peptide binding to the signal peptide of VEGF

<400>  34

Ser Pro Ser Thr Ser Ser Arg
1               5


<210>  35
<211>  7

<212>  PRT
<213>  Artificial Sequence

<220>
<223>  Peptide binding to the signal peptide of VEGF

<400>  35

Thr Leu Ser His Thr Arg Thr
1               5


<210>  36
<211>  7
<212>  PRT
<213>  Artificial Sequence

<220>
<223>  Peptide binding to the signal peptide of VEGF

<400>  36

Phe Pro Ala Thr Ala Ala Ser
1               5


<210>  37
<211>  7
<212>  PRT
<213>  Artificial Sequence

<220>
<223>  Peptide binding to the signal peptide of VEGF

<400>  37

Glu Pro Ala Ser Ser Arg His
1               5


<210>  38
<211>  7
<212>  PRT
<213>  Artificial Sequence

<220>
<223>  Peptide binding to the signal peptide of VEGF

<400>  38

Ser Leu Thr Gly Pro Ile Ala
1               5


<210>  39
<211>  7
<212>  PRT
<213>  Artificial Sequence

<220>
<223>  Peptide binding to the signal peptide of VEGF

<400>  39

Leu Ala Phe Tyr Ala Cys Phe
1               5


<210>  40
<211>  7

<212>  PRT
<213>  Artificial Sequence

<220>
<223>  Peptide binding to the signal peptide of VEGF

<400>  40

Asp Tyr Pro Trp Pro Leu Pro
1               5


<210>  41
<211>  7
<212>  PRT
<213>  Artificial Sequence

<220>
<223>  Peptide binding to the signal peptide of VEGF

<400>  41

Pro Leu His Pro Pro Gln Pro
1               5


<210>  42
<211>  7
<212>  PRT
<213>  Artificial Sequence

<220>
<223>  Peptide binding to the signal peptide of VEGF

<400>  42

Leu Pro Arg Pro Leu His Ser
1               5


<210>  43
<211>  7
<212>  PRT
<213>  Artificial Sequence

<220>
<223>  Peptide binding to the signal peptide of VEGF

<400>  43

Val Ser Gly Ser Ser Pro Gln
1               5


<210>  44
<211>  7
<212>  PRT
<213>  Artificial Sequence

<220>
<223>  Peptide binding to the signal peptide of VEGF

<400>  44

Pro Ser Leu Phe Pro Ser Met
1               5


<210>  45
<211>  7

<212>  PRT
<213>  Artificial Sequence

<220>
<223>  Peptide binding to the signal peptide of VEGF

<400>  45

Glu Ala Ser Ser Ser Arg Ala
1               5


<210>  46
<211>  7
<212>  PRT
<213>  Artificial Sequence

<220>
<223>  Peptide binding to the signal peptide of VEGF

<400>  46

Ser Arg Ser Ala Leu Glu Ser
1               5


<210>  47
<211>  7
<212>  PRT
<213>  Artificial Sequence

<220>
<223>  Peptide binding to the signal peptide of VEGF

<400>  47

Pro Ser Ala Leu Ala Phe Tyr
1               5


<210>  48
<211>  7
<212>  PRT
<213>  Artificial Sequence

<220>
<223>  Peptide binding to the signal peptide of VEGF

<400>  48

Asn Ala Ser Arg Thr Thr Leu
1               5


<210>  49
<211>  7
<212>  PRT
<213>  Artificial Sequence

<220>
<223>  Peptide binding to the signal peptide of VEGF

<400>  49

His Ala Leu Pro Ala Pro Asp
1               5


<210>  50
<211>  183

<212>  DNA
<213>  Artificial Sequence

<220>
<223>  DNA sequence encoding an N-terminal fragment of VEGF

<400>  50
atgaactttc tgctgtcttg ggtgcattgg agccttgcct tgctgctcta cctccaccat       60

gccaagtggt cccaggctgc acccatggca gaaggaggag ggcagaatca tcacgaagtg      120

gtgaagttca tggatgtcta tcagcgcagc tactgccatc accatcacca tcacgtggtg      180

acc                                                                    183


<210>  51
<211>  60
<212>  PRT
<213>  Artificial Sequence

<220>
<223>  N-terminal fragment of VEGF

<400>  51

Met Asn Phe Leu Leu Ser Trp Val His Trp Ser Leu Ala Leu Leu Leu
1               5                   10                  15


Tyr Leu His His Ala Lys Trp Ser Gln Ala Ala Pro Met Ala Glu Gly
            20                  25                  30


Gly Gly Gln Asn His His Glu Val Val Lys Phe Met Asp Val Tyr Gln
        35                  40                  45


Arg Ser Tyr Cys His His His His His His Val Val
    50                  55                  60

## Description

The present invention relates to a method for identifying an inhibitor of protein translocation.

Signal peptides, also named signal sequences or leader peptides, drive the translocation of proteins destined to undergo the secretory pathway (secreted or transmembrane proteins) to the endoplasmic reticulum in eukaryotes or across the cytoplasmic membrane in bacteria. When newly synthesized nascent chains containing a signal peptide emerge from the ribosome, the signal peptide is recognized by the signal recognition particle (SRP) which guides the ribosome-nascent chain complex to the translocation channel ("translocon") embedded in the membrane of the endoplasmic reticulum or the bacterial cytoplasmic membrane. The translocon is an aqueous pore, formed by the heterotrimeric Sec61 complex composed of the α-, β- and γ-subunits. This complex is also called Sec61p in eukaryotes, and SecY in prokaryotes. The translocation complex is evolutionarily highly conserved. The transfer of the ribosome-nascent chain complex from the SRP to the translocon occurs via binding of the ribosome-nascent chain-SRP complex to the SRP receptor (SR) adjacent to the Sec61 translocon, followed by attachment of the ribosome-nascent chain complex to the translocon. Another signal-peptide-dependent step is the "gating", the opening of the translocation channel towards the luminal side of the endoplasmic reticulum, allowing passage of the newly synthesized nascent chain into the endoplasmic reticulum. In eukaryotes, most translocation events occur co-translationally, although also post-translational translocation is possible. In bacteria, post-translational translocation is mediated via SecA through SecY in the cytoplasmic bacterial membrane.

The translocon also mediates integration of transmembrane domains into the membrane of the ER and is required for the insertion of single-spanning membrane proteins (type I and type II, signal-anchors) but also complex polytopic membrane proteins. In case of polytopic membrane proteins, coordinated insertion of transmembrane domains allows correct assembly and folding. Orientation of the transmembrane domain is also dependent on charges of amino-acid residues adjacent to or within the transmembrane domain.

Signal peptides, signal sequences or leader peptides, usually of 20 to 30 amino-acid residues in length, frequently located at the amino-terminal end of a protein, have a typical three-domain structure, representing a frequently positively charged n-region, a hydrophobic core region (h-region) and a more polar c-region frequently containing a site for signal peptide cleavage. Based on these features of signal peptides, bio-informatic approaches have been developed which allow the prediction of signal peptides and their cleavage sites fairly reliable for different organisms. Apart from this characteristic three-domain structure, signal peptides represent a high diversity within their primary sequence which has a relatively high degree of conservation across different species.

A transmembrane domain is defined by a stretch of typically 20 to 30 hydrophobic amino-acid residues, but can be also longer or shorter. A stretch of apolar residues can be also interrupted by polar amino-acid residues.

Inhibitors of the process of cotranslational translocation have been described. Examples are the compounds HUN-7293, its derivates CAM741 or NFI028, and "cotransin" which can inhibit the process of cotranslational translocation of human vascular cell adhesion molecule-1 (VCAM-1) and, in case of CAM741, that of human vascular endothelial growth factor (VEGF; VEGF-A) to the endoplasmic reticulum while not affecting translocation of various other proteins. This inhibition is dependent on the signal peptide sequences of VCAM-1 and VEGF. The exact target of HUN-7293, its derivatives CAM741 or NFI028, or cotransin and whether these compounds can directly bind to the signal peptides of VCAM-1 and/or VEGF is currently not known. A HUN-7293 derivative has been shown to bind to the Sec61 translocon which now appears to be the site of compound action.

Another example is apratoxin A which can also inhibit the process of cotranslational translocation. Insertion of synthetic signal peptides into membranes has also been shown to block the process of cotranslational translocation. Sterols have also been shown to inhibit cotranslational translocation. However, all these inhibitors are not acting in a substrate-specific manner. A recent publication demonstrated that eeyarestatin I has the capability to block the process of cotranslational translocation by preventing the transfer of the nascent polypeptide chain to the Sec61 translocon (Cross et al., J. Cell. Sci. 122 (2009): 4393-4400) .

A cellular screening assay for identification of compounds inhibiting the process of cotranslational translocation has been disclosed in WO 02/057778. Therein the use of a signal peptide fused to a reporter protein and its expression in mammalian cells for screening purposes is disclosed. Limitations of cellular screening systems may be the identification of compounds with off-target effects and the presence of various different potential targets within the secretory pathway in living cells.

It is an object of the present invention to provide means and methods for identifying compounds which are able to bind to translocation signal and transmembrane domains of proteins and polypeptides utilizing the extreme diversity of the primary sequences of such domains. The compounds identified should be able to block specifically the translocation of selected proteins through biological membranes and pores. Such compounds may be able to bind to and alter the properties of such domains in a way that the normal translocation process is compromised but may be also useful to improve such a process where inefficient translocation occurs.

The present invention relates to a method for identifying an inhibitor of protein translocation comprising the steps of:
a) providing a nucleic acid molecule encoding a polypeptide comprising a translocation signal domain and/or a transmembrane domain and having no stop codon in the reading frame of said polypeptide,
b) translating *in vitro* the nucleic acid molecule of step a) into a ribosome bound nascent polypeptide by contacting said nucleic acid molecule with an in vitro translation system comprising ribosomes,
c) optionally contacting the ribosome bound nascent polypeptide of step b) with a compound releasing the polypeptide from the ribosome and isolating said released polypeptide,
d) incubating at least one test compound or a library of test compounds
   i) with the ribosome bound nascent polypeptide of step b) or optionally
   ii) with the isolated polypeptide of step c),
e) isolating the ribosome bound nascent polypeptide or optionally isolated polypeptide of step d) and identifying the test compound bound to said polypeptide.

Signal peptides or transmembrane domains can mediate the transport of nascent polypeptides from the translating ribosome or after termination of translation through the translocation machinery to the endoplasmic reticulum in mammals, insects, parasites, fungi or across the cytoplasmic membrane in bacteria, or mediate transport of viral proteins in different host organisms. The present invention relates to the use of DNA or RNA molecules encoding polypeptides containing a signal peptide and/or transmembrane domain in such a screening assay. This screening method aims at the identification of compounds, e.g. peptides, binding specifically and selectively to signal peptides and/or transmembrane domains of a nascent polypeptide. This process can be generalized and is applicable to polypeptides generated by different types of organisms, or polypeptides encoded by viral DNA or RNA.

During translation of RNA, a "nascent" polypeptide emerges from the translating ribosome. During this process, such polypeptide can only acquire a limited degree of protein folding. A transmembrane domain can acquire protein folding, such as formation of an α-helix, and in some cases the transmembrane segment can be folded already inside the ribosome. A limited degree of folding, such as formation of α-helical structures, is also likely for signal peptides.

Due to the hydrophobicity of signal peptides and/or transmembrane domains, the use of a synthetic peptide as bait would require experimental conditions which can mimic a membrane and/or ribosomal environment (e.g. by use of proteoliposomes or lipid micelles) which may not reflect the correct environment of a nascent polypeptide when emerging from the ribosome. The present invention overcomes this problem by using nascent chains directly translating on a ribosome as a bait.

The method according to the present invention allows to identify compounds which specifically bind to specific translocation signal domains and transmembrane domains. The identification of such compounds is particularly advantageous, because with such compounds it is possible to target specific proteins destined to be translocated and to prevent or enhance their secretion through or integration into a cell membrane.

In a first step of the method of the present invention a nucleic acid molecule is provided which encodes for a polypeptide comprising a translocation signal and/or transmembrane domain. These domains can be identified by software based methods or are already known in the art.

The nucleic acid molecule does not comprise a stop codon in the reading frame of the encoded polypeptide. Due to the lack of such a stop codon the nascent polypeptide synthesized in step b) remains bound to the ribosome in the form of peptidyl-tRNAs forming a polypeptide/ribosome complex. This complex can be isolated by simple purification steps including, for instance, centrifugation, preferably ultra-centrifugation. Such a centrifugation step can be performed with a relative centrifugation force of at least 100,000 x g, preferably with a relative centrifugation force of at least 200,000 x g, more preferably with a relative centrifugation force of at least 350,000 x g, most preferably with a relative centrifugation force of at least 500,000 x g up to 550,000 x g, for at least 5 min, preferably for at least 15 min, more preferably for at least 30 min. The speed of the centrifugation as well as the time employed for the centrifugation can be varied and to some extent depends on each other. If the speed of the centrifuge is increased the centrifugation time can be reduced and vice versa.

In an optional step of the method of the present invention the preferably isolated ribosome bound nascent polypeptide is contacted with a substance capable to release the polypeptide from the ribosome. This is of advantage if a domain of the polypeptide or protein should become accessible which may be otherwise buried within the ribosome.

The nucleic acid molecule employed in step a) of the method of the present invention is preferably RNA. However, it is of course also possible to use DNA. If DNA is used, then the *in vi*tro translation system should also comprise compounds allowing the transcription of the DNA into RNA (including amongst others a DNA dependent RNA polymerase).

According to a preferred embodiment of the present invention the polypeptide comprising the translocation signal domain and/or the transmembrane domain comprises 40 to 200, preferably 40 to 150, more preferably 40 to 100, even more preferably 40 to 90, in particular 40 to 80, amino-acid residues.

When a nascent polypeptide is synthesized on the ribosome, approximately 35 to 40 amino acid residues remain within the ribosome while the amino-terminal signal peptide or transmembrane domain is exposed at the ribosomal exit tunnel. Therefore the polypeptide encoded by the nucleic acid molecule used in the present method comprises preferably at least 40 amino acid residues.

According to a further preferred embodiment of the present invention the polypeptide comprising the translocation signal domain and/or the transmembrane domain comprises further a purification tag.

A purification tag has the advantage that the ribosome-bound or free polypeptide comprising the translocation signal domain and/or the transmembrane domain can be bound to, for instance, a solid support in order to isolate the polypeptide or to immobilize the whole ribosome-nascent chain complex. This is particularly advantageous when the nascent polypeptide bound to the ribosome is released therefrom (e.g. by adding substances like puromycin to the polypeptide/ribosome complex) or to isolate the whole ribosome-polypeptide complex. According to a preferred embodiment it is also possible to use the purification tag to isolate the polypeptide comprising the translocation signal domain and/or the transmembrane domain bound to the inhibitor.

The purification tag which is preferably fused to the polypeptide is preferably selected from the group consisting of a HIS-tag, a calmodulin-binding peptide-tag, a myc-tag, a FLAG-tag, a strep-tag, HA-tag or any other tag known in the art. Such a purification tag may be located at different sites in the polypeptide, most preferably at the carboxy-terminal end of the polypeptide, but may be also located at the amino-terminal end of the signal peptide and/or transmembrane domain optionally separated from the signal peptide and/or transmembrane domain by a spacer region, or may be located shortly after the signal peptide or transmembrane sequence.

Purification tags are well known in the art. The most preferred purification tags comprise less than 100, preferably less than 80, more preferably less than 50, amino acid residues in order not to influence the binding of the test compounds to the polypeptide comprising the translocation signal domain and/or the transmembrane domain. However, if said polypeptide remains after translation bound to the ribosome, said purification tag or at least a major part thereof is located within the ribosome and not exposed to test compounds if located at the carboxy-terminal end.

According to a preferred embodiment of the present invention the compound releasing the polypeptide from the ribosome or to dissociate the ribosome is selected from the group of puromycin, high-salt (500 mM KCl)/puromycin, RNAse and ethylenediaminetetraacetic acid (EDTA).

In the course of protein translation the finalised protein or polypeptide is released from the ribosome by compounds as listed above or by the provision of a stop codon present on the nucleic acid molecule encoding said polypeptide.

The *in vitro* translation system is preferably selected from the group consisting of a wheat germ lysate, a reticulocyte lysate, a bacterial *in vitro* translation system, yeast cell lysate, mammalian cell lysate and insect cell lysate. The most preferable system would be wheat germ lysate due to low content of SRP which may mask the signal peptide sequence and prevent binding of a test compound or library.

*In vitro* translation systems to be used according to the present invention are well known in the art. These systems comprise components which, in combination with a DNA or RNA template (linear and/or circular) encoding a polypeptide of interest, allow *in vitro* translation of the polypeptide. Such systems typically comprise possibly an RNA-polymerase, ATP, GTP, CTP, UTP among other factors for transcription and amino acids, and a cell extract containing ribosomes capable of supporting *in vitro* translation. The template is a DNA or RNA molecule comprising a gene encoding the desired polypeptide. The DNA molecule can be obtained, for instance, by PCR. A transcription reaction of an RNA molecule from a DNA molecule as template which may be cloned into a vector containing an RNA polymerase promoter or generated by PCR fused to an RNA polymerase promoter, is also well known in the art. Transcription of the DNA molecule can occur *in vivo* or *in vitro,* from prokaryotic or eukaryotic cells or cell extracts, prior to *in vitro* translation. *In vitro* transcription systems for both prokaryotic and eukaryotic systems are commercially available and well known in the art. *In vitro* translation systems are made from prokaryotic cells such as E. *coli,* or from eukaryotic cells such as rabbit reticulocyte, wheat germ lysate, or insect cell lysates.

Transcription and translation can of course also occur simultaneously in a coupled *in vitro* transcription/translation system, wherein the template contains appropriate regulatory elements, such as a promoter and a ribosome binding site. Also such systems are well known in the art and are available for both eukaryotic and prokaryotic applications.

Cell extracts, which can be used for translation reactions alone, or for both transcription and translation reactions, contain all the enzymes and factors necessary to carry out the intended reactions. Suitable cell extracts for prokaryotic and eukaryotic translation systems are known in art. Examples include prokaryotic lysates such as E. coli lysates, and eukaryotic lysates such as wheat germ extracts, insect cell lysates, reticulocyte lysates, oocyte lysates and human cell lysates.

The test compound which is examined for its potential activity as protein translocation inhibitor is preferably a peptide, polypeptide, but may be also a natural product or derivatives thereof, or a library of natural products, a synthetic peptide with non-natural amino acid residues or other derivatizations such as non-peptidic backbones, or peptidic sequences from combinatorial chemical libraries.

Next to peptides and polypeptides it is of course also possible to use other substances, such as chemical compounds or a library of compounds, as test compounds. Therefore in the method of the present invention all possible substances can be tested.

According to a preferred embodiment of the present invention the library of test compounds is a peptide display library (linear or cyclic peptides), but may be also a small molecule library, a combinatorial library, or a library of chemical compounds.

It is particularly preferred to use a peptide surface display system (e.g. phage peptide surface display, bacterial peptide surface display, baculovirus peptide surface display system, yeast or mammalian cell peptide surface display systems, ribosome display or any related methodology) to screen for peptides (e.g. cyclic or linear peptides) binding to sequences of a nascent polypeptide translating on a ribosome, in particular to signal peptides and/or transmembrane domains and sequences adjacent to signal peptides and/or transmembrane domains of polypeptides. Linear or cyclic peptides which bind to signal peptides, transmembrane domains or other sequences of a nascent polypeptide may serve as structural candidates for pharmaceutical development to inhibit the process of cotranslational translocation and/or transmembrane domain insertion into the membrane of the endoplasmic reticulum in eukaryotes or bacterial membranes or transport to extracellular space by bacteria or may be useful to enhance these processes in conditions where such processes are perturbed. Another application of this screening method includes the identification of peptides which are useful for correction of misfolded proteins. Another application is the biotechnological use, such as modulation of protein production, folding, secretion and/or membrane protein insertion *in vitro* or by different organisms.

The test compound or single compounds of the test compound library may potentially bind to parts of the *in vitro* translation system. This is particularly the case when ribosome bound nascent polypeptides are tested. Although ribosome bound nascent polypeptides are preferably purified in order to remove undesired components of the *in vitro* translation system, the ribosomes of said system are still part of the complex formed in the course of the *in vitro* translation. Since single members of the test compound library may potentially bind unspecifically to these ribosomes, it is advantageous to include a subtractive selection step. A subtractive selection step is a step, which removes compounds with undesired properties. In the method according to the present invention a subtractive selection step is effected in order to remove compounds binding to ribosomes, for instance. If compounds binding to ribosomes are not removed and used in the method according to the present invention, compounds are identified which bind to ribosomes and not to the target polypeptide.

The subtractive selection step can be exemplarily performed as follows. In order to select compounds specific for a polypeptide comprising a translocation signal domain and/or a transmembrane domain one could first select those compounds which bind to those ribosomes used to produce the ribosome bound nascent polypeptides. Thereby the compounds are contacted with the ribosomes and the compound/ribosome complexes formed are removed from the solution comprising the multiplicity of compounds and ribosomes (e.g. by using a centrifuge or by immobilization on solid support). The supernatant obtained is substantially free of compounds binding to ribosomes and is contacted with the ribosome bound nascent polypeptides allowing the isolation of those compounds which bind to the polypeptides.

It is also possible to use a ribosome translating an unrelated peptide, optionally containing a signal peptide or transmembrane domain, for the subtraction step.

It is also possible to use a polypeptide lacking a signal peptide or transmembrane domain for the subtraction step.

Therefore at least one test compound or the library of test compounds is preferably subjected to a subtractive selection step prior step d) effected to remove test compounds capable of binding to the substances present in the *in vitro* translation system, in particular to the ribosomes of said translation system.

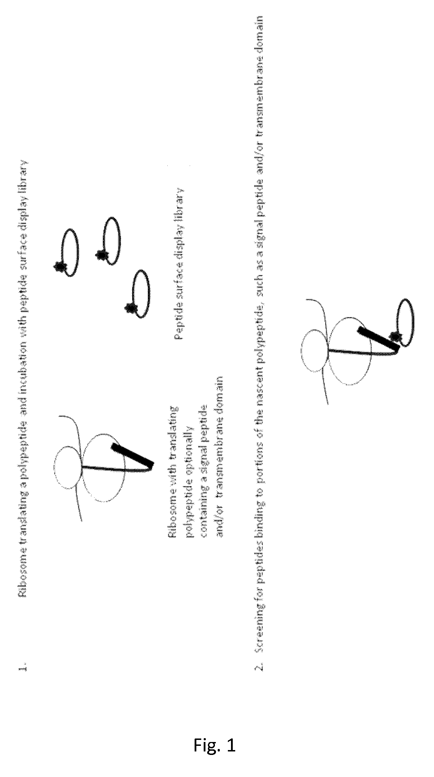

In one aspect the present invention provides the use of a ribosome translating a nascent polypeptide which may contain signal peptides and/or transmembrane domains including signal anchors in an *in vitro* translation system (i.e. wheat germ lysate, reticulocyte lysate, bacterial *in vitro* translation system, yeast or mammalian or insect cell lysates or any related method) for a screening process as shown in Fig. 1 to identify compounds, preferably linear or cyclic peptides, binding to sequences of the nascent polypeptide, comprising the following steps including
a. the use of DNA or RNA to generate a nascent polypeptide on a translating ribosome,
b. the use of a peptide surface display library (i.e. phage peptide surface display, bacterial peptide surface display, baculovirus peptide surface display, eukaryotic cell peptide surface display (e.g. yeast, mammalian), ribosome peptide display, or any related method) for such a screening process to identify linear or cyclic peptides binding to a nascent polypeptide on a translating ribosome (e.g. by the use of transcripts lacking a stop codon) or after termination of translation (i.e. through presence of a stop codon, release of nascent polypeptide by chemicals such as puromycin, RNAse, EDTA, or any related method),
c. the enrichment and isolation of bound peptides by co-sedimentation of peptides presented by phage, bacteria, baculovirus or eukaryotic cell or ribosomes used for peptide display with ribosomes translating the bait nascent polypeptide or after termination of translation,
d. the enrichment and isolation of bound peptides by binding the bait polypeptide to a matrix which recognizes a tag present in the bait polypeptide (i.e. HA-tag, His-tag, Strep-tag or other tags) or through any endogenous sequence in the bait polypeptide which is recognized by specific antibodies or antisera or similar method or any related methodology,
e. the enrichment and isolation of bound peptides by immobilization of ribosomes translating the bait polypeptide on a solid support either through immobilization of the ribosome on a solid support or through immobilization of translating ribosomes through features of the translated polypeptide (i.e. a tag present in the bait polypeptide, or by a modified amino acid residue in the translated polypeptide),
f. the subtraction of the peptide surface display library with non-translating ribosomes or ribosomes translating an unrelated polypeptide, or ribosomes translating a polypeptide lacking a signal peptide or transmembrane domain,
g. the repeated panning of the peptide surface display library against the bait polypeptide translating on a ribosome or after termination of translation,
h. the isolation of peptides bound to the bait polypeptide and sequencing of the encoding DNA or RNA or protein,
i. the use of the displayed peptide identified in steps a.-h. in context with the producing host (i.e. phage, bacteria, virus, cells or ribosome) in a binding assay for determining the interaction of the displayed peptide with the bait polypeptide (i.e. co-precipitation or co-sedimentation of displayed peptide and bait polypeptide, immobilization of displayed peptide and/or bait polypeptide and determination of binding to each other, by biochemical methods such as binding determined by surface plasmon resonance (SPR), Western blot analysis using specific antibodies or antisera, enzyme linked immunosorbent assay (ELISA)-based methods, mass spectrometry and/or nuclear magnetic resonance (NMR), labelling of one and/or the binding partner by radiolabelling, fluorescence labelling, fluorescence resonance energy transfer (FRET) technologies or any related methodology, two-hybrid methodology or any related methodologies, chemical crosslinking, analysis of binding by quantification of the nucleic acid encoding the peptide, such as by PCR, RT-PCR, quantification by PCR using specific primers, optionally by analysis of PCR products by gel electrophoresis or quantification in the presence of an intercalating dye such as SYBR-Green, determination of melting curves, determination of high-resolution melting curves, or quantification of nucleic acids encoding the peptide sequence by Real-Time PCR using dye-labelled probes),
j. the analysis of a displayed peptide identified in steps a.-h. for inhibition or enhancement of targeting and/or translocation, protease protection, signal peptide cleavage, N-glycosylation, folding, protein secretion or transmembrane domain insertion by *in vitro* methods (e.g. *in vitro* translocation in the presence of microsomal membranes or cell extracts or isolated bacterial inner membranes) or in living cells (e.g. analysis of protein translocation in living cells by pulse-chase labelling experiments, protease protection, signal peptide cleavage, N-glycosylation, folding such as disulphide bond formation, analysis of biological activity of protein, expression and/or secretion in cells transfected with a plasmid-DNA encoding the bait protein or analysis of expression and/or secretion of endogenously produced protein in eukaryotic cells or bacteria),
k. the analysis of a synthetic linear or cyclic peptide containing sequences identified through the screening process in a binding assay (i.e. co-precipitation or co-sedimentation of synthetic peptide and bait polypeptide, immobilization of synthetic peptide and/or bait polypeptide and determination of binding to each other, by biochemical methods such as binding determined by SPR, ELISA-based methods, mass spectrometry and/or NMR, labelling of one and/or the binding partner by radiolabelling, fluorescence labelling, FRET technologies or any related methodology, two-hybrid methodology or any related methodologies or chemical crosslinking),
l. the analysis of a synthetic linear or cyclic peptide containing sequences identified through the screening process for inhibition or enhancement of targeting and/or translocation, protease protection, signal peptide cleavage, N-glycosylation, folding, protein secretion or transmembrane domain insertion by *in vitro* methods (e.g. *in vitro* translocation in the presence of microsomal membranes or cell extracts or membrane preparations or isolated bacterial inner membranes) or in living cells (e.g. analysis of protein translocation in living cells by pulse-chase labelling experiments, protease protection, signal peptide cleavage, N-glycosylation, folding such as disulphide bond formation, analysis of biological activity of protein, expression and/or secretion in cells transfected with a plasmid-DNA encoding the bait protein or analysis of expression and/or secretion of endogenously produced protein in eukaryotic cells or bacteria or cells transfected with plasmid DNA expressing a fusion of the signal peptide or transmembrane protein and a reporter protein (see e.g. WO 02/057778)),
m. selecting a candidate peptide or lead peptide which can bind to the bait polypeptide identified in steps a.-j. or a.-l.,
n. further development of a peptide identified by such a screening process for pharmaceutical purposes or biotechnological applications and/or as structural basis for a low-molecular weight compound and/or chemical derivatization.

Means for such processes may be included in an assay or a kit.

Sequences of polypeptides may be obtained from DNA and/or protein databases. Sequences encoding signal peptides and/or transmembrane proteins may be obtained from DNA and/or protein databases or by using software for prediction of signal peptides and/or transmembrane domains. In general, a polypeptide sequence may be generated based on DNA-sequences available in databases using conventional cloning techniques, genomic or cDNA library screening, polymerase chain reaction or gene synthesis.

Appropriate RNA may be generated by *in vitro* transcription using conventional transcription systems, transcription/translation systems or gene synthesis.

Constructs lacking a stop codon may be generated by restriction digestion of plasmid-DNA, by generation of DNA by polymerase chain reaction or gene synthesis. A sequence encoding a tag may be introduced into the DNA construct by standard cloning techniques, polymerase chain reaction, mutagenesis or gene synthesis.

Sedimentation of translating ribosomes may be achieved by ultracentrifugation through a layer of sucrose solution or a related method.

Release of nascent chains bound to ribosomes may be achieved using chemicals such as puromycin, high-salt-conditions, EDTA or biologicals such as RNase.

Immobilization of tagged nascent chains may be achieved by an appropriate system such as Ni-NTA for binding of 6xHis-tags, streptavidin with strep-tags, anti-HA antibody or antiserum with HA-tags, anti-Myc antibodies or antiserum with myc tags, or by binding to an antibody or antiserum to another domain of the bait polypeptide.

An appropriate peptide surface display system may be a phage peptide surface display system using filamentous phage such as M13, a bacterial peptide surface display system, a baculovirus, yeast or other eukaryotic cellular peptide surface display systems or ribosome peptide display methodology.

Bound peptides may be released from the translating nascent chains by elution at high salt concentrations, by using chelators, by competing with the bait polypeptide, by using biotin with streptavidin-bound bait polypeptide, at low pH or by using a reducing agent to enrich for cyclic peptides. Amplification of peptide candidates may be achieved by expansion of the host in an appropriate system, such as amplification of phages in a host bacterial strain, or amplification of a baculovirus in an insect cell. Bacteria presenting peptides on their surface may be expanded in appropriate culture medium.

DNA or RNA from phages, bacteria or cells may be isolated according to standard techniques and sequencing performed either directly using appropriate sequencing primers or after amplification of the DNA of interest by polymerase chain reaction. RNA may be transcribed into DNA by reverse transcription.

Analyses of targeting and/or translocation may be performed using *in vitro* translation or *in vitro* transcription/translation systems and determination of protein translocation into endoplasmic reticulum or bacterial inner membranes (e.g. by the use of microsomal membranes or preparations of bacterial inner membranes) and determination of translocated protein by protection from proteases (e.g. Proteinase K or other proteases), determination of signal peptide cleavage where appropriate, determination of N-glycosylation where appropriate, determination of folding (e.g. formation of disulphide bonds or through activity of a protein) where appropriate using *in vitro* methodologies.

Analyses of cotranslational translocation in living cells may be achieved by pulse-chase labeling experiments and analysis of protection from proteases (e.g. Proteinase K or other proteases), determination of signal peptide cleavage where appropriate, determination of N-glycosylation where appropriate, determination of folding (e.g. formation of disulphide bonds or through biological activity of a protein) where appropriate, expression of protein on cell surface and/or release of protein by cells by standard methods such as Western blot analysis using an appropriate antibody or antiserum, ELISA-based methods, labeling (such as radiolabelling, fluorescence labelling, or any related methodology) .

Peptide binding may be determined by co-precipitation or co-sedimentation, by biochemical methods such as binding determined by SPR, ELISA-based methods, mass-spectrometry and/or NMR, labelling of one and/or the binding partner by radiolabelling, fluorescence labelling, FRET technologies or any related methodology, two-hybrid methodology or any related methodologies or chemical crosslinking, analysis of binding by quantification of the nucleic acid encoding the peptide, such as by PCR, RT-PCR, quantification by PCR using specific primers, optionally by analysis of PCR products by gel electrophoresis or quantification in the presence of an intercalating dye such as SYBR-Green, determination of melting curves, determination of high-resolution melting curves, or quantification of nucleic acids encoding the peptide sequence by Real-Time PCR using dye-labelled probes.

Another aspect of the present invention relates to a peptide identified by such as screening process using vascular endothelial growth factor-A (VEGF-A; VEGF) short nascent chains containing the signal peptide of VEGF-A as bait having the amino acid sequence PLHARLP (SEQ ID NO:1), FLFRLF (SEQ ID NO:2), PMTSPPS (SEQ ID NO:3), NPISLHL (SEQ ID NO:4), TASSVKL (SEQ ID NO:5), SVSGSPS (SEQ ID NO:6), SGSSPWH (SEQ ID NO:7), HPSSTHQ (SEQ ID NO:8), NLSSSWI (SEQ ID NO:9), LTRPSPS (SEQ ID NO:10), SPSLLPA (SEQ ID NO:11), SSPLLGR (SEQ ID NO:12), PVAPSTH (SEQ ID NO:13), LGALSHT (SEQ ID NO:14), SPRPSLS (SEQ ID NO:15), PANFGPS (SEQ ID NO: 16), LTLPNRT (SEQ ID NO: 17), AFPGMKA (SEQ ID NO: 18), LPPLQRL (SEQ ID NO: 19), PPPFLPL (SEQ ID NO: 20), EIRTPSV (SEQ ID NO: 21), HAWTFNS (SEQ ID NO: 22), SYPLRPP (SEQ ID NO: 23), LPVSSSS (SEQ ID NO: 24), LALSAAR (SEQ ID NO: 25), SLNSDLF (SEQ ID NO: 26), SPHLIPS (SEQ ID NO: 27), HPMFPLS (SEQ ID NO: 28), HPMFWPI (SEQ ID NO: 29), WASQAHP (SEQ ID NO: 30), SSTLHPS (SEQ ID NO: 31), HALSSRT (SEQ ID NO: 32), PHTRGFM (SEQ ID NO: 33), SPSTSSR (SEQ ID NO: 34), TLSHTRT (SEQ ID NO: 35), FPATAAS (SEQ ID NO: 36), EPASSRH (SEQ ID NO: 37), SLTGPIA (SEQ ID NO: 38), LAFYACF (SEQ ID NO: 39), DYPWPLP (SEQ ID NO: 40), PLHPPQP (SEQ ID NO: 41), LPRPLHS (SEQ ID NO: 42), VSGSSPQ (SEQ ID NO: 43), PSLFPSM (SEQ ID NO: 44), EASSSRA (SEQ ID NO: 45), SRSALES (SEQ ID NO: 46), PSALAFY (SEQ ID NO: 47), NASRTTL (SEQ ID NO: 48), and/or HALPAPD (SEQ ID NO: 49).

VEGF-A, VEGF or vascular permeability factor (VPF), plays an essential role in angiogenesis, and can induce endothelial cell proliferation, migration, and tube formation. VEGF is also implicated in several diseases such as tumor angiogenesis, diabetic retinopathy, age-related macular degeneration, and psoriasis. Six isoforms of VEGF (121, 145, 165, 183, 189 and 206 amino-acid residues) are generated by alternative mRNA splicing but all contain the same 26 amino acid-residue signal peptide.

The peptides of the present invention are able to bind to said signal peptide and, hence, can be used to inhibit VEGF release by mammalian cells. A precedence for a successful interference with this cellular pathway has been demonstrated by the compound CAM741 which can inhibit the process of cotranslational translocation of VEGF-A in a signal-peptide-dependent manner and which also inhibits VEGF-A release in living cells (Harant, et al., Mol Pharmacol 71 (2007): 1657-1665).

The binding of a peptide to the signal peptide of VEGF-A could alter the properties in a way that it is not further recognized as signal peptide by the translocation apparatus.

Examples provided in the literature have shown that only few amino-acid substitutions within a signal peptide sequence can result in a failure of normal translocation (Kim et al., Dev. Cell 2 (2002): 207-217)

Such a compound or a derivative thereof may be used for topical treatment or applied via inhalation, but also oral or inhaled for systemic administration. The peptides may be able to penetrate cellular membranes or modified by chemical derivatization to enhance intracellular delivery. Alternatively, the peptides may be delivered using a formulation sufficient for cell penetration to allow intracellular delivery of the peptide. Such a formulation may be a peptide bioconjugate, or by using liposomes or nanoparticles for peptide delivery.

According to a preferred embodiment of the present invention the peptides comprise at their N- and/or C-terminus at least one cysteine residue. This allows to cyclisize the peptides or to bind said peptides to another substance. This may enhance intracellular delivery of said peptides, such as by coupling to hydrophobic amino-acid residues or peptides, such as cell-penetrating peptides, lipids, sterols or other bioconjugates. The coupling to other substances can occur via the free cysteine. The presence of a cysteine residue may not be a prerequisite as cyclization may be also introduced by chemical modification.

Since the peptides of the present invention are able to prevent the release of VEGF from cells, said peptides are suited for treating or preventing a VEGF-associated disease.

The VEGF-associated disease is preferably selected from the group consisting of tumor, diabetic retinopathy, age-related macular degeneration, rheumatoid arthritis, inflammation, psoriasis and asthma.

Another aspect of the present invention relates to the use of a peptide according to the present invention for manufacturing a medicament for treating or preventing a VEGF-associated disease.

According to a preferred embodiment of the present invention the VEGF-associated disease is selected from the group consisting of tumor, diabetic retinopathy, age-related macular degeneration, rheumatoid arthritis, inflammation, psoriasis and asthma.

For analysis of the biological activity of said peptides, their binding to the VEGF signal peptide may be determined. Inhibition of the process of cotranslational translocation may be determined by in vitro methods such as targeting and/or translocation to microsomal membranes. Biological activity in living cells may be analysed in cells transfected with an VEGF-A expression plasmid and inhibition of VEGF-A release determined in the absence or presence of various concentration of peptide. Analysis of biological activity of the peptides can be further determined in cells expressing VEGF-A endogenously, such as cell lines or primary cells, either untreated or under conditions where expression and release of VEGF-A is increased, such as stimulation with a factor or under hypoxic conditions. Finally, inhibition of VEGF-A may be determined by in vivo angiogenesis or tumor models.

The present invention is further illustrated in the following figures and examples, however, without being restricted thereto.

Fig. 1 shows a schematic representation of the screening process. A nascent chain on a translating ribosome is incubated with a peptide surface display library for enrichment and isolation of peptide sequences binding to nascent polypeptide chains.

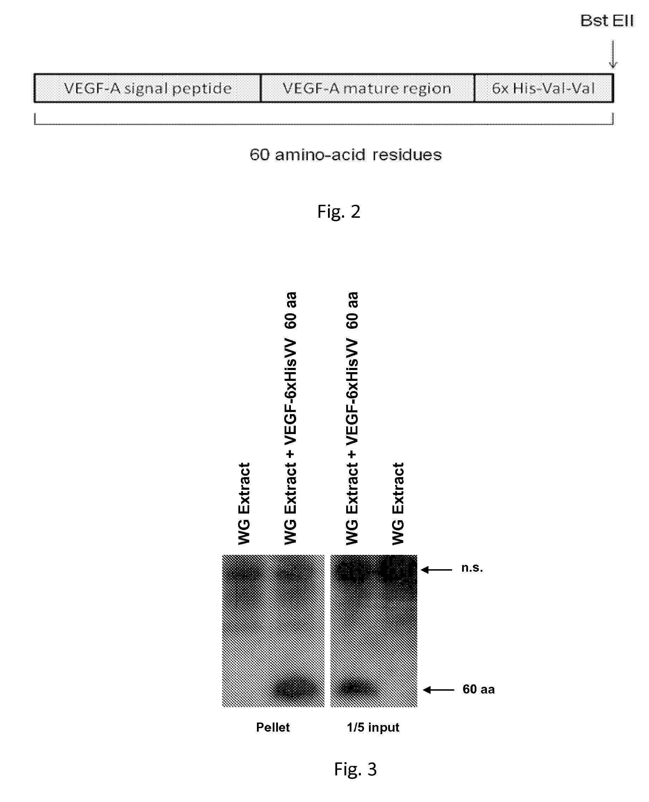

Fig. 2 shows the DNA construct used in the examples for screening a peptide surface display library against short VEGF-A nascent chains. The DNA construct encodes the amino-terminal 26 amino-acid-residue signal peptide of VEGF-A, 26 amino-acid-residues of the VEGF-A mature region and a tag consisting of six histidine residues followed by two valine residues and no stop codon upstream of a BstEII restriction site.

Fig. 3 shows the *in vitro* translated VEGF-A truncated nascent chains using wheat germ lysate or wheat germ lysate alone after sedimentation of the ribosomes by ultracentrifugation (left panel). The right panel shows 1/5 of the input of the translation reaction for ultracentrifugation. The translation products were resolved by SDS-polyacrylamide gel electrophoresis followed by Western blot and detection using an anti-PentaHis antibody (Qiagen).

### EXAMPLES:

The following examples describe the identification of inhibitors which are useful for the inhibition of VEGF release.

### Example 1: CONSTRUCTION OF PLASMID-DNA

A DNA encoding the 52 amino-terminal amino acid residues of human VEGF-A plus a carboxy-terminal tag consisting of six histidine residues followed by two valine residues was cloned into a plasmid vector containing elements for in vitro transcription and eukaryotic cell expression. The construct contained sequences encoding the 26 amino-terminal signal peptide of VEGF-A, 26 amino-acid residues of its mature domain and the C-terminal tag consisting of six histidine residues followed by two valine residues without a stop codon upstream of a BstEII restriction site comprising the DNA sequence 
ATGAACTTTCTGCTGTCTTGGGTGCATTGGAGCCTTGCCTTGCTGCTCTACCTCCACCATGCCA
AGTGGTCCCAGGCTGCACCCATGGCAGAAGGAGGAGGGCAGAATCATCACGAAGTGGTGAAGTT
CATGGATGTCTATCAGCGCAGCTACTGCCATCACCATCACCATCACGTGGTGACC (BstEII
site underlined)
 allowing expression of a 60 amino-acid-residue polypeptide chain comprising the polypeptide sequence MNFLLSWVHWSLALLLYLHHAKWSQAAPMAEGGGQNHHEVVKFMDVYQRSYCHHHHHHVV (signal peptide sequence is underlined)as shown in FIG. 2. Constructs were linearized by restriction digestion with BstEII, purified and used as template for transcription into RNA using the RiboMAX Large Scale RNA Production System (Promega).

### Example 2: SCREENING PROCEDURE - METHOD 1

RNA encoding parts of the VEGF-A coding region (amino-terminal 52 amino-acid residues of VEGF plus six histidine residues and two valine residues as in example 1) was subjected to *in vitro* translation using wheat germ lysate (Promega). Due to lack of a stop codon the nascent polypeptide remained attached to the ribosomes (stalled ribosomes with nascent chains associated as peptidyl-tRNAs). The translation reaction was applied on top of a 0.5 M sucrose solution, and ribosomes sedimented by ultracentrifugation through the layer of 0.5 M sucrose solution in a S100AT3 rotor at 90,000 rpm for 20 min or in a S140AT rotor at 100,000 rpm for 30 min using a M120SE Discovery ultracentrifuge (Thermo Scientific). The result from this process is shown in FIG. 3. A M13 phage peptide surface display library (Ph.D.™-C7C Disulfide Constrained Peptide Library Kit; New England Biolabs) was used for screening. The library contained phages displaying random disulfide-constrained heptapeptide sequences expressed at the amino-terminus of the M13 pIII minor coat protein. Five microliter (7.5 x 10¹⁰ pfu) of the phage library were pre-incubated with the sedimented stalled ribosomes associated with VEGF-A nascent chains for 30 min at room temperature. The ribosomes and phages were then co-sedimented through a 0.5 M sucrose solution in a S100AT3 rotor at 90,000 rpm for 20 min. The co-sedimented ribosomes and phages were used for titration and amplification of the phage population in the bacterial host strain E. *coli* ER2738(Step I). The amplified phages were then incubated with non-translating ribosomes (=subtraction) for 30 min at room temperature, followed by ultracentrifugation through a layer of a 0.5 M sucrose solution in a S100AT3 rotor at 90,000 rpm for 20 min (Step II). The supernatant was amplified in the bacterial host strain E. *coli* ER2738 and used again for panning against the short VEGF nascent chains on translating ribosomes (Steps III), followed by another subtraction step (Step IV) and a final panning step against the target (Step V).

Isolation of single phage plaques and sequencing revealed a DNA sequence encoding the heptapeptide PLHARLP (SEQ ID NO:1) flanked by the two cysteine residues of the M13 pIII minor coat protein.

### Example 3: SCREENING PROCEDURE - METHOD 2

RNA encoding parts of the VEGF-A coding region (amino-terminal 52 amino-acid residues plus six histidine residues and two valine residues as in example 1) was subjected to *in vitro* translation using wheat germ lysate (Promega). Due to the lack of the stop codon the nascent polypeptide remained attached to the ribosomes. The translation reaction was applied on top of a 0.5 M sucrose solution and ribosomes sedimented by ultracentrifugation through the layer of 0.5 M sucrose solution in a S100AT3 rotor using a Discovery M120SE centrifuge (Thermo Scientific) at 90,000 rpm for 20 min. Five microliter (7.5 x 10¹⁰ pfu) of the disulfide constrained peptide library (Ph.D.™-C7C Disulfide Constrained Peptide Library Kit; New England Biolabs) were incubated with Ni-NTA magnetic agarose beads (Qiagen) for 30 min at room temperature and Ni-NTA-bound phages separated from unbound phages using a magnetic stand (= subtraction). The supernatant was used for further screening. Sedimented ribosomes were incubated with the subtracted Ph.D.™-C7C Constrained Peptide Library for 30-45 min in phosphate-buffered saline followed by release of the nascent polypeptide chains from the ribosome by treatment with puromycin at 30°C for 20 min. The released nascent chain-phage complex was incubated with Ni-NTA magnetic agarose beads for 30-45 min at room temperature. After four washes with phosphate-buffered saline, bound phages were eluted with 10 mM dithiothreitol to enrich cyclic peptides and subjected to titration and amplification in the bacterial host strain E. *coli* ER2738. After three additional rounds of panning, single phage plaques were isolated and phage DNA subjected to sequencing. The following sequences encoding peptide sequences flanked by the two cysteine residues of the M13 minor coat protein pIII were identified: one hexapeptide FLFRLF (SEQ ID NO:2), and the heptapeptides PMTSPPS (SEQ ID NO:3), NPISLHL (SEQ ID NO:4), TASSVKL (SEQ ID NO:5), SVSGSPS (SEQ ID NO:6), SGSSPWH (SEQ ID NO:7), HPSSTHQ (SEQ ID NO:8), NLSSSWI (SEQ ID NO:9), LTRPSPS (SEQ ID NO:10), SPSLLPA (SEQ ID NO:11), SSPLLGR (SEQ ID NO:12), PVAPSTH (SEQ ID NO:13), LGALSHT (SEQ ID NO:14), SPRPSLS (SEQ ID NO:15).

### Example 4: BINDING OF PHAGES TO BAIT POLYPEPTIDE

Isolated phages from the screening methods 1 and 2 as in examples 2 and 3 were amplified in 10 ml cultures of the bacterial host strain E. *coli* ER2738 and phages prepared by sedimentation with polyethylene glycol/sodium chloride. Phages were then resuspended in Tris-buffered saline and kept frozen at -20°C in the presence of 50% glycerol.

Parts of the VEGF-A coding region (amino-terminal 52 amino acid residues plus six histidine residues and two valine residues as in example 1) were subjected to *in vitro* translation using wheat germ lysate (Promega). As negative control, a second wheat germ reaction was prepared adding all components except RNA. Both reactions were applied separately on top of a 0.5 M sucrose solution and ribosomes sedimented by ultracentrifugation through the layer of 0.5 M sucrose solution in a S100AT3 rotor using a Discovery M120SE centrifuge (Thermo Scientific) at 90,000 rpm for 20 min. Sedimented ribosomes from the VEGF-A translation reaction or the negative control were incubated with the isolated phages at room temperature for 30-45 min in phosphate-buffered saline followed by release of the nascent polypeptide chains from the ribosome by treatment with puromycin at 30°C for 20 min. The released nascent chain-phage complex was incubated with Ni-NTA magnetic agarose beads for 30-45 min at room temperature. After four washes with phosphate-buffered saline, the bound phages were eluted with 10 mM dithiothreitol and subjected to titration in the bacterial host strain E. *coli* ER2738. The number of plaques was counted on the next day.

### Example 5: SCREENING PROCEDURE - METHOD 3

In this example the immobilization of a protein-ribosome complex on a solid support is shown.

RNA encoding parts of the VEGF-A coding region (amino-terminal 52 amino-acid residues plus six histidine residues and two valine residues as in examples 1-4) was subjected to *in vi*tro translation using wheat germ lysate (Promega) in the presence of Transcend tRNA (precharged ε-labelled biotinylated lysine tRNA-complex; Promega). Due to the lack of the stop codon the nascent polypeptide remained attached to the ribosomes. The translation reaction was applied on top of a 0.5 M sucrose solution and ribosomes sedimented by ultracentrifugation through the layer of 0.5 M sucrose solution in a S100AT3 rotor using a Discovery M120SE centrifuge (Thermo Scientific) at 90,000 rpm for 20 min. Five microliter (7.5 x 10¹⁰ pfu) of the disulfide constrained peptide library (Ph.D.™-C7C Disulfide Constrained Peptide Library Kit; New England Biolabs) were added to the sedimented stalled ribosomes with VEGF-A nascent chains and incubated in streptavidin-coated microwells for 60 min at room temperature. The wells were washed four times with phosphate-buffered saline, and bound phages eluted with 10 mM DTT for 20 min. The eluted phages were then subjected to titration and amplification in the bacterial host strain E. *coli* ER2738 (Step I). For subtraction, non-translating ribosomes were incubated with the amplificate from Step I and incubated in streptavidin-coated microwells for 180 min. at room temperature to subtract for streptavidin- binding phages and non-specific ribosome-binding phages (Step II). The amplificate from this step was used for panning against VEGF-A-translating ribosomes as in step I, except with an incubation time of 120 min (Steps III and IV). The amplificate from step IV was then again added to the streptavidin-coated microwells to subtract streptavidin-binding phages. Phages from steps III and IV were isolated and phage DNA subjected to sequencing. In another setting, the phage amplificate from step III was used for panning against VEGF-A-translating ribosomes in streptavidin-coated microwells as in step I with an incubation time for 120 min. Wells were then washed twice with phosphate-buffered saline followed by an incubation of the wells with RNAse ONE (Promega) for 30 min at 37°C to dissociate ribosomes. This latter step was included to remove any non-specific ribosome-binding phages. The wells were then washed again with phosphate-buffered saline for four times and bound phages eluted with 10 mM DTT. Phages were then subjected to titration and amplification, and DNA from isolated phage plaques subjected to sequencing.
The following sequences encoding peptide sequences flanked by the two cysteine residues of the M13 minor coat protein pIII were identified: PANFGPS (SEQ ID NO: 16), LTLPNRT (SEQ ID NO: 17), AFPGMKA (SEQ ID NO: 18), LPPLQRL (SEQ ID NO: 19), PPPFLPL (SEQ ID NO: 20), EIRTPSV (SEQ ID NO: 21), HAWTFNS (SEQ ID NO: 22), SYPLRPP (SEQ ID NO: 23), LPVSSSS (SEQ ID NO: 24), LALSAAR (SEQ ID NO: 25), SLNSDLF (SEQ ID NO: 26), SPHLIPS (SEQ ID NO: 27), HPMFPLS (SEQ ID NO: 28), HPMFWPI (SEQ ID NO: 29), WASQAHP (SEQ ID NO: 30), SSTLHPS (SEQ ID NO: 31), HALSSRT (SEQ ID NO: 32), PHTRGFM (SEQ ID NO: 33), SPSTSSR (SEQ ID NO: 34), TLSHTRT (SEQ ID NO: 35), FPATAAS (SEQ ID NO: 36), EPASSRH (SEQ ID NO: 37), SLTGPIA (SEQ ID NO: 38), LAFYACF (SEQ ID NO: 39), DYPWPLP (SEQ ID NO: 40), PLHPPQP (SEQ ID NO: 41), LPRPLHS (SEQ ID NO: 42), VSGSSPQ (SEQ ID NO: 43), PSLFPSM (SEQ ID NO: 44), EASSSRA (SEQ ID NO: 45), SRSALES (SEQ ID NO: 46), PSALAFY (SEQ ID NO: 47), NASRTTL (SEQ ID NO: 48), and HALPAPD (SEQ ID NO: 49).

## Claims

1. Method for identifying an inhibitor of protein translocation comprising the steps of:
a) providing a nucleic acid molecule encoding a polypeptide comprising a translocation signal domain and/or a transmembrane domain and having no stop codon in the reading frame of said polypeptide,
b) translating *in vitro* the nucleic acid molecule of step a) into a ribosome bound nascent polypeptide by contacting said nucleic acid molecule with an *in vitro* translation system comprising ribosomes,
c) optionally contacting the ribosome bound nascent polypeptide of step b) with a compound releasing the polypeptide from the ribosome and isolating said released polypeptide,
d) incubating at least one test compound or a library of test compounds
i) with the ribosome bound nascent polypeptide of step b) or optionally
ii) with the isolated polypeptide of step c),
e) isolating the ribosome bound nascent polypeptide or optionally isolated polypeptide of step d) and identifying the test compound bound to said polypeptide.

2. Method according to claim 1, **characterised in that** the polypeptide comprising the translocation signal domain and/or the transmembrane domain comprises 40 to 200, preferably 40 to 150, more preferably 40 to 100, even more preferably 40 to 90, in particular 40 to 80, amino acid residues.

3. Method according to claim 1 or 2, **characterised in that** the polypeptide comprising the translocation signal domain and/or the transmembrane domain further comprises a purification tag.

4. Method according to claim 3, **characterised in that** the purification tag is selected from the group consisting of a HIS-tag, a calmodulin-binding peptide-tag, a myc-tag, a FLAG-tag, a strep-tag, or a HA-tag.

5. Method according to any one of claims 1 to 4, **characterised in that** the compound releasing the polypeptide from the ribosome is selected from the group of puromycin, RNAse and ethylenediaminetetraacetic acid (EDTA).

6. Method according to any one of claims 1 to 5, **characterised in that** the *in vitro* translation system is selected from the group consisting of a wheat germ lysate, a reticulocyte lysate, a bacterial *in vitro* translation system, yeast cell lysate, mammalian cell lysate and insect cell lysate.

7. Method according to any one of claims 1 to 6, **characterised in that** the test compound is a peptide, polypeptide, a natural product or derivatives thereof, a synthetic peptide with non-natural amino acid residues or other derivatizations such as non-peptidic backbones.

8. Method according to any one of claims 1 to 7, **characterised in that** the library of test compounds is a peptide surface display system, a small molecule library, a combinatorial library, a peptide library or a library of natural products.

9. Method according to any one of claims 1 to 8, **characterised in that** the at least one test compound or the library of test compounds is subjected to a subtractive selection step prior step d) effected to remove test compounds capable of binding to the substances present in the *in vitro* translation system, in particular to the ribosomes of said translation system.

10. Peptide having the amino acid sequence PLHARLP (SEQ ID NO:1), FLFRLF (SEQ ID NO:2), PMTSPPS (SEQ ID NO:3), NPISLHL (SEQ ID NO:4), TASSVKL (SEQ ID NO:5), SVSGSPS (SEQ ID NO:6), SGSSPWH (SEQ ID NO:7), HPSSTHQ (SEQ ID NO:8), NLSSSWI (SEQ ID NO:9), LTRPSPS (SEQ ID NO:10), SPSLLPA (SEQ ID NO:11), SSPLLGR (SEQ ID NO:12), PVAPSTH (SEQ ID NO:13), LGALSHT (SEQ ID NO:14) and/or SPRPSLS (SEQ ID NO:15), PANFGPS (SEQ ID NO: 16), LTLPNRT (SEQ ID NO: 17), AFPGMKA (SEQ ID NO: 18), LPPLQRL (SEQ ID NO: 19), PPPFLPL (SEQ ID NO: 20), EIRTPSV (SEQ ID NO: 21), HAWTFNS (SEQ ID NO: 22), SYPLRPP (SEQ ID NO: 23), LPVSSSS (SEQ ID NO: 24), LALSAAR (SEQ ID NO: 25), SLNSDLF (SEQ ID NO: 26), SPHLIPS (SEQ ID NO: 27), HPMFPLS (SEQ ID NO: 28), HPMFWPI (SEQ ID NO: 29), WASQAHP (SEQ ID NO: 30), SSTLHPS (SEQ ID NO: 31), HALSSRT (SEQ ID NO: 32), PHTRGFM (SEQ ID NO: 33), SPSTSSR (SEQ ID NO: 34), TLSHTRT (SEQ ID NO: 35), FPATAAS (SEQ ID NO: 36), EPASSRH (SEQ ID NO: 37), SLTGPIA (SEQ ID NO: 38), LAFYACF (SEQ ID NO: 39), DYPWPLP (SEQ ID NO: 40), PLHPPQP (SEQ ID NO: 41), LPRPLHS (SEQ ID NO: 42), VSGSSPQ (SEQ ID NO: 43), PSLFPSM (SEQ ID NO: 44), EASSSRA (SEQ ID NO: 45), SRSALES (SEQ ID NO: 46), PSALAFY (SEQ ID NO: 47), NASRTTL (SEQ ID NO: 48), and/or HALPAPD (SEQ ID NO: 49)

11. Peptide according to claim 10, **characterised in that** at the N- and/or C-terminus of the peptide at least one cysteine residue is provided.

12. Peptide according to claim 10 or 11 for treating or preventing a VEGF-associated disease.

13. Peptide according to claim 12, **characterised in that** the VEGF-associated disease is selected from the group consisting of tumor, diabetic retinopathy, age-related macular degeneration, rheumatoid arthritis, inflammation, psoriasis and asthma.

14. Use of a peptide according to claim 10 or 11 for manufacturing a medicament for treating or preventing a VEGF-associated disease.

15. Use according to claim 14, **characterised in that** the VEGF-associated disease is selected from the group consisting of tumor, diabetic retinopathy, age-related macular degeneration, rheumatoid arthritis, inflammation, psoriasis and asthma.
